# EUROPEAN PATENT APPLICATION

(11) **EP 2 005 967 A1**
(43) Date of publication of application: **24.12.2008**
(21) Application number: 07380263.9
(22) Date of filing: 27.09.2007
(51) Int. Cl.: A61K 38/22, A61P 3/04

(54) **Use of leptin in the treatment of alterations in food habits**

(30) Priority: 20.06.2007 ES 200701707
(71) Applicant: Universitat de les Illes Balears, Palma de Mallorca 07122 (ES)
(72) Inventor: Palou March, Mariona, 07122 Palma de Mallorca (ES); Tobaruela Arbona, Aixa, 07122 Palma de Mallorca (ES)
(74) Representative: ABG Patentes, S.L.

(57) **Abstract**

The present invention is addressed to the use of leptin, a fragment thereof or a mimetic compound of leptin action, in the production of a preparation for consumption to administer to lactating mammals, in particular humans, for the treatment and/or prevention of alterations in food habits, in particular, to treat and/or prevent the acquisition of undesirable food preferences, to regulate appetite, and other related disorders as a consequence of alterations in leptin sensitivity.

## Description

### Field of the invention

The present invention is related to the prevention or treatment of alterations in food behaviour. In particular it refers to the regulation of food preferences by carbohydrate-rich foods instead of fat-rich foods, as well as the improvement of appetite control regulation and other disorders as a result of an improvement of the leptin response.

### State of the Art

Body weight excess is an ever more frequent disorder in our developed societies and, in fact, it has become an important problem not only because it is an aesthetic problem, but also because it is a health problem and a source of other social and economic problems. Body weight excess is related to a higher risk of developing a wide range of complications such as respiratory and cardiac illnesses, diabetes, hypertension, etc ...

In general, the changes in lifestyle in developed countries, particularly a higher intake of fat associated to lower physical activity, have been considered one of the main causes of the rise in the incidence of metabolic disorders. The consumption of a hyper caloric diet and a greater fat intake is related to an increase in the adipose tissue mass, and an increase in adipose tissue size is translated into a higher release of non-sterified fatty acids (NEFA) into the blood; said fatty acids compete with other fuels such as glucose for their utilization as a substrate in skeletal muscle. This results in a decrease in the use of glucose by this tissue and, thus, an increase in the concentration of circulating glucose. On the other hand, a higher ectopic storage of lipids in the liver and in skeletal muscle has also been related to lower insulin sensitivity.

Therefore, the modulation of food behaviour, including both control of appetite, and food preferences in favour of less fatty foods with a lower caloric content, can provide a mechanism for the prevention of the development of metabolic disorders (Langley-Evans et al., Matern Child Nutr., 1, 142-148, 2005), particularly when food with a high caloric density is widely available, as happens in our developed societies. Other mechanisms, different from the control of appetite or food preferences, genetic or acquired, may be responsible for a higher or lower predisposition for the development, for instance, of diabetes and related disorders.

One of the more important signals playing a part in the maintenance of the energy balance and so of body weight is leptin, a circulating protein codified by the *ob* gene which is mainly expressed in the adipose tissue. Leptin plays a central role in the regulation of energy balance, inhibiting food intake and increasing energy waste (Zhang et al., Nature, 372, 425-432, 1994). This protein circulates in blood in a concentration that is quite proportional to the size of the fat depots; it passes through the haematoencephalic barrier by means of a saturable system, and exerts most of its effects on energy balance at a central level, afterwards the interaction of the protein with receptors located in hypothalamic neurons and in other regions of the brain (Tartaglia et al., Cell. 83, 1263-1271, 1995).

Animals with defects in the leptin route, because they do not produce the functional protein or because they express defective forms of its receptor, are characterised by hyperphagia and massive obesity of early appearance, as well as by suffering diabetes, hypothermia and infertility. In humans, congenic defects in the route of leptin (lack of leptin or of its receptor) are also related to morbid obesity of early appearance (Clement et al., Nature, 392, 398-401, 1998; Montague et al., Nature, 387, 903-908, 1997; Strobel et al., Nat. Genet., 18, 213-215, 1998). In this sense, the use of leptin in the treatment or prevention of diabetes mellitus (WO97/02004) whose direct cause is obesity was proposed.

Nevertheless, although it was thought that the short-term anorexigenic role of leptin could contribute to controlling the problem of obesity and metabolic disorders in obese people, unfortunately, leptin administration has not been proven to be effective in the vast majority of cases of obesity in humans, due to the fact that these show resistance to the action of this protein.

It is known that, with age, the circulating levels of leptin increase (Li H. Matheny et al., Diabetes 1997, 46, 2035-9; Iossa S. et al., J Nutr. 1999, 129, 1593-6) and there is an impairment in sensitivity to this hormone (Qian H. et al., Proc Soc Exp Biol Med 1998, 219, 160-5; Scarpace PJ. et al., Neuropharmacology, 2000, 39, 1872-9). However, although the concentration of circulating leptin is usually considered to be proportional to body fat mass and this mass usually increases as we grow old, there is evidence that the increase in leptinemia and the development of leptin resistance with age occurs, at least in part, independently of the increase in adiposity (Gabriely I. et al., Diabetes, 2002, 51, 1016-21).

Although the adipose tissue is the main source, leptin is also produced by other tissues, including the placenta, stomach and mammary epithelium, and it is also present in maternal milk. Several studies show that breastfeeding, in comparison with bottle-feeding (infant formula milks), is related with a lower risk of suffering different complications in adulthood (Armstrong and Reilly, Lancet. 359, 2003-2004, 2002; Gillman et al., Jama., 285, 2461-2567, 2001; Kramer, J. Pediatr., 98, 883-887, 1981; von Kries et al., Bmj., 319, 147-150, 1999). Maternal milk has a lot of hormones and bioactive peptides that may play an important role in the development of the neonate, and could be responsible for exerting these effects of "metabolic imprinting" during this critical period of development. The fact that leptin is present in maternal milk, together with the results in rats and humans (Miralles et al., Obesity, 14, 1371-1377, 2006; Pico et al., Int. J. Obes., 2007), point to leptin as one of the bioactive compounds that could be responsible for the beneficial effects of breastfeeding and the lower medium/long-term incidence in the prevention of overweight and obesity.

However, to date no effect has been demonstrated of the intake of additional leptin during breastfeeding on the regulation or modulation of food habits in later stages of life or, in particular, in adulthood, as would be the case of a better appetite control or preference for less fatty foods, and, related to this, a higher sensitivity to leptin, which would considerably decrease the risk of suffering, in the short- and long-term, metabolic and medical complications, such as obesity, diabetes and other illnesses or disorders related to the impairment of leptin sensitivity.

### Brief description of the invention

The authors of this invention have surprisingly found that the administration of additional leptin to that acquired through maternal milk, in lactating mammals, makes it possible to modulate food behaviour towards a healthier diet by improving leptin sensitivity during adulthood. In particular, the administration of leptin during lactation has been demonstrated to allow the regulation of both appetite and food preferences in subsequent ages, decreasing the tendency to consume fat-rich and high energy foods, and promoting, instead, the consumption, among others, of carbohydrates. This fact will significantly contribute to protecting from the subsequent development of metabolic and medical complications of the utmost importance.

This new application of leptin is based on research carried out by the inventors on experimental animals, in which they found an appetite reduction, associated with an improvement in the sensitivity to this leptin and with a reduction in leptin levels (leptinemia), as well as a reduction in the preference for the intake of fat-rich foods and an increase in the intake of carbohydrates in adulthood, when these animals had been treated with an additional amount of leptin during their lactation.

The observed effect of reduction of leptinemia and the maintenance or non-deterioration of leptin sensitivity in maturity, brought about by supplementation with leptin during lactation, could also be interesting effects from a clinical point of view. Hence, high leptin circulating levels have been associated in humans with an increase in the risk of cardiovascular disease [Ren, J., J. Endocrinol., 2004, 181, 1-10] and development of insulin resistance [Huang, KC, et al., Int. J. Obes. Relat. Metab. Disord., 2004, 28, 470-5], and this even independently of body mass index/adiposity. Moreover, high levels of circulating leptin may favour the development of resistance to the anorexigenic effects of this hormone, which leads to perpetuating the development and maintenance of obesity and/or its complications. In fact, there is evidence suggesting that, in rats, leptin resistance would be the main determinant of body weight increase and age-related adiposity [Iossa, S. et al., J. Nutr., 1999, 129, 1593-6].

In this way, an aspect of the present invention is related to the use of leptin, a fragment thereof or a mimetic product of leptin action, in the production of a preparation for consumption to administer to lactating mammals, in particular lactating humans, for the treatment and/or prevention of alterations in food habits during adulthood.

In another aspect, the invention is related to a method for the treatment and/or prevention of an alteration in food behaviour in adulthood, this method comprises the administration to a lactating mammal of an effective amount of leptin, a fragment thereof or a mimetic product of leptin action.

In one particular aspect of the invention, the alteration of food habits consists of the acquisition of undesirable food preferences, in particular, a preference for the intake of fat-rich and high-energy food. More particularly, it is a preference for foods rich in saturated fats.

In another particular aspect, the alteration in food habits leads to an appetite alteration, in particular this alteration is an excess of appetite.

In another aspect, the invention is related to the use of leptin, a fragment of leptin or a mimetic product of leptin action, in the production of a preparation for consumption to administer to lactating mammals, in particular lactating humans, for the treatment and/or prevention of the deterioration of leptin sensitivity, of a deficient action of endogenous leptin or of alterations that are accompanied by a deficient sensitivity or response to the administration of exogenous leptin, in subsequent ages or in adulthood.

In another aspect, the invention is directed towards a method for the treatment and/or prevention of the deterioration of leptin action, of a deficient action of endogenous leptin or of alterations that are accompanied by a deficient sensitivity or response to the administration of exogenous leptin, in adulthood, or, in general, in ages subsequent to lactation; said method comprises the administration to a lactating mammal of an effective amount of leptin, a fragment thereof or a mimetic product of leptin action.

In another particular aspect, leptin, a fragment thereof or a mimetic product of leptin action, is administered to lactating humans and to domestic lactating animals. In a preferential embodiment, it is administered to lactating humans.

In another particular aspect, leptin, a fragment thereof or a mimetic product of leptin action is also administered to lactating mothers and pregnant females, at higher doses than the ones planned for the lactating neonates, with the purpose that, via the mother, sufficient leptin will arrive to foetuses or lactating neonates.

Finally, in an additional aspect, the preparation for consumption is a pharmaceutical composition, a food composition or a nutritional supplement.

### Brief figure description

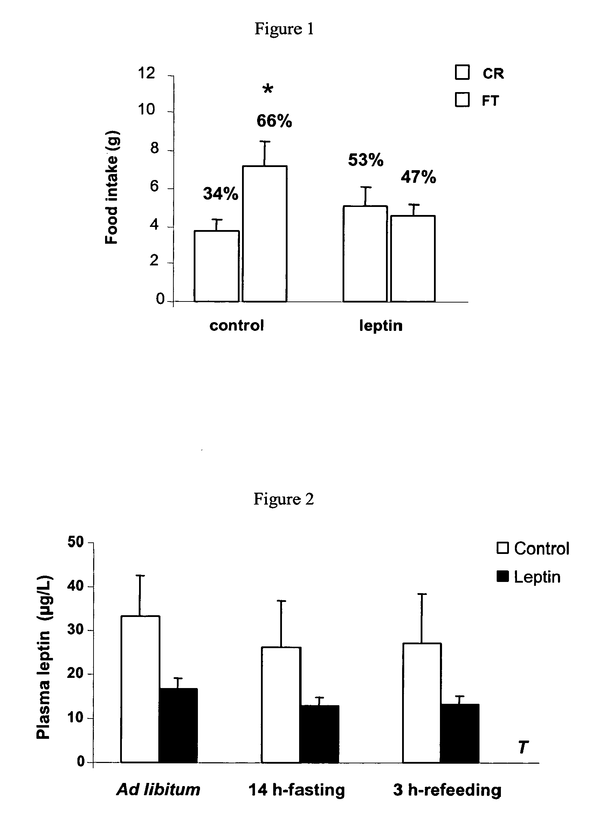

**Figure 1****:** Dietary preferences for a carbohydrate-rich diet (CR) or a fat-rich diet (FR) measured by the two-bottle test in 8-month-old male rats from control group and leptin-treated during lactation group. Results are expressed as means ± SEM for the values of ingestion from 3 different days. * Statistical significance of the value of the intake of FR *νs* the value of the intake of CR (p<0.05, Student's t test).
**Figure 2****:** Serum leptin concentration under different feeding conditions *(ad libitum, 14* hours fasting and 3 hours pair-fed refeeding after 14 hours fasting) in 9-month-old male Wistar rats which received a daily oral dose of leptin during lactation. Results are expressed as means ± SEM of 6 animals per group. T, effect of leptin treatment (p<0.05 two way ANOVA).

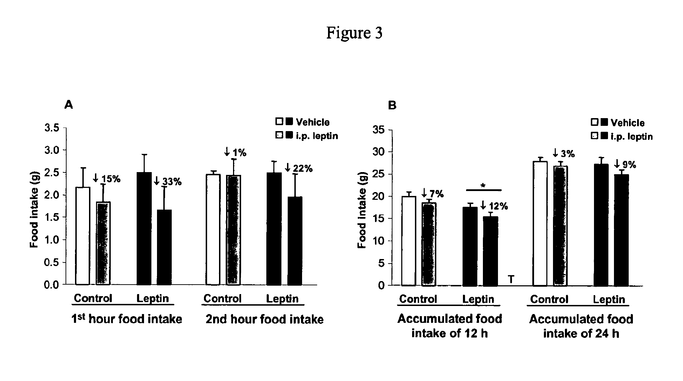

Figure 3: Food intake after the leptin resistance test made in adult male Wistar rats that received a daily oral dose of leptin or vehicle during lactation. Food intake was measured after the intraperitoneal administration of leptin (2mg/kg) (i.p leptin) or saline (vehicle) just before lights off at 20:00 h. Bars represent the mean value of the food intake during the 1^{st} and 2^{nd} hour after the intraperitonael leptin/vehicle administration (A), and the cumulative food intake 12 and 24 hours after the intraperitoneal leptin/vehicle administration (B). The Percentages of the reduction in food intake as effect of the intraperitoneal leptin administration respect to their controls injected with saline are indicated. * effect of intraperitoneal leptin administration (p<0.05, test de la t de Student).

### Detailed description of the invention

In the context of the present invention, by leptin we understand the protein itself without modification, or rather, its definition may also include complete chain polypeptides of leptin which, in turn, may include modified molecules that contain adducts such as dextran, fatty acids or pegylated groups, or biologically active fragments or mimetic compounds of leptin action.

Leptin polypeptides can be isolated from physiological sources or can be produced in a recombinant way. In a preferential aspect of the present invention, leptin is a recombinant leptin that can come from different species, including humans, and it can be a whole or partially hydrolysed recombinant leptin. The term "partially hydrolysed leptin" refers to any fragment of this molecule that conserves the functional properties of leptin. The recombinant polypeptides may be generated in any expression system, such as yeast expression systems, bacterian from insects or mammals or other animals, and may be produced with or without their naturally-occurring secretion signal peptides. Adequate leptin fragments that can be used in the present invention include those described in US 6,187,751, WO97/46585 and WO00/11173.

Likewise, different methods for cloning and purifying leptin compounds suitable for use in the present invention have been described in scientific literature [Pelleymounter et al., Science 269: 540-546 (1995); Campfield et al., Science 269: 546-549, (1995), and Chehab et al., Nat. Gen., 12: 318-320 (1996)].

For their part, the mimetic products of leptin action refer to those active agents that share one or more biological activities with leptin. They may be naturally-occurring polypeptides that share biological activities with leptin, such as the human obesity protein homolog-1 (WO01/25428), or the ciliary neurotrophic factor or axokine [Lambert et al., Proc. Natl. Acad. Sci., 98:4652-4657 (2001) and WO98/22128].

Alternatively, a mimetic product of leptin action may be a polypeptide of recombinant leptin which has replacements, eliminations and insertions of amino acids relative to a native leptin sequence, which substantially retains or has a boosted leptin biological activity.

Also alternatively, a mimetic product of leptin action may be a drug based on a small synthetic molecule, a peptide or a polypeptide that is able to exert one or more of the biological effects of leptin.

In one particular embodiment, natural or recombinant leptin or the fragments of leptin used in the present invention are derived from the same species as those that are going to be treated or prevented. Therefore, humans will preferably be treated with human leptin. However, leptin can be used to treat and/or prevent species different from the ones the aforesaid comes from. In a preferential embodiment, leptin is obtained from humans, dogs, cats, mice, apes, pigs or other bovine or sheep sources. Nevertheless, other forms of leptin may be used for the treatment and/or prevention of other species. For example, bovine, ovine, caprine, sow, buffalo, etc. leptin may be used to prevent or treat humans.

In the field of the present invention, by *"alterations in food habits"* it is understood those adverse alterations that lead to the acquisition of undesirable food preferences (such as a higher preference for fat, in particular for saturated fat), and also appetite alterations.

By *"undesirable food preferences"* it is understood those preferences developed by animals or humans that cause the excessive consumption of hypercaloric and high-energy density foods, such as those with a high fat-content (mainly of animal source) and sugars, potentially responsible for metabolic disorders such as obesity and diabetes.

By *"appetite alterations"* it is understood the development of a repetitive wish for food and/or drink that leads to an excessive food intake.

By *"lactating mammal",* it is understood any mammal, and includes, although it is not limited to, domestic animals, rodents, primates and humans, in particular a human, in his/her first months of life. Preferentially, this lactating mammal is a human or a domestic animal, such as a dog or a cat, or a farm animal, such as a sheep or a cow. In a more preferential embodiment, lactating mammal is a human being, male or female, of any race. However, this definition would also include humans up to the age of 3-4 years.

The aforementioned alterations in food habits can be caused as a consequence of deterioration in leptin sensitivity that occurs with age and, to a certain extent, with body fat mass, due to an increase in the circulating levels of this hormone. This decrease in sensitivity or response to leptin occurs both for endogenous leptin and for leptin administered in an exogenous way.

In consequence, in another aspect, the invention is related to the use of leptin, a fragment thereof or a mimetic product of leptin action, in the production of a preparation for consumption to administer to lactating mammals, in particular lactating humans, for the treatment and/or prevention of the deterioration of leptin sensitivity, of a deficient action of endogenous leptin or of alterations related to a deficient sensitivity or response to exogenous leptin administration, in adulthood.

By *"alterations that are accompanied by a deficient sensitivity to leptin"* it is understood any condition or alteration of the organism that determines or brings with it a *"deficient action of leptin"* whether it be endogenous or exogenous, that is, a condition in which leptin effects (for example the appetite inhibitor effect or anorexigenic effect of leptin or other effects related with the control of appetite and energy waste, or effects on different functions related to reproduction, the immune system, hematopoyesis, angiogenesis or other cellular functions) are lower than the norm or those considered a referent.

The "deterioration of leptin sensitivity" is any decrease or loss of the capacity of the organism to produce a physiological response to leptin, that is, it leads to a deficient leptin action, whether it is endogenous-produced leptin or exogenous-administered leptin.

Although adequate feeding and nutrition are important at all life stages, these are particularly important during childhood. Undesirable dietary habits can begin from birth or at a few months or years of life and can be established in a more or less persistent way in other stages, such as around puberty, with a tendency to be consolidated throughout the whole life. Childhood is, for this reason, a crucial period to act on food behaviour and on the mechanisms that determine its implementation, especially if this implementation lasts in posterior stages, because habits acquired at this stage are going to be determinant for the health of the future adult. By this reason, it is of particular relevance to prevent the appearance of these undesirable dietary habits form early ages, even from the lactating period and even, as far as possible, from a few months before birth.

Thus, in a preferential aspect of the present invention, the use of leptin is especially addressed at administration to lactating neonates, in a way that healthy habits in their feeding over a short- and long-term can be generated.

Leptin administration can also be administered to a lactating neonate directly or through the mother's milk, particularly when leptin levels in maternal milk are insufficient. For this reason, in another particular embodiment of the invention, the use of leptin, a fragment thereof or a mimetic product of leptin action is particularly aimed at administration to lactating mothers. In this way, it is possible to increase leptin concentration in maternal milk, with the lactating neonate receiving an additional amount of this protein.

Likewise, leptin administration to a pregnant female mammal could favourably influence the metabolic pathways imposed to her descendant. Therefore, in another particular embodiment of the invention, the use of leptin, a fragment thereof or of a mimetic product of leptin action is particularly aimed at administration to pregnant women, with the aim of preventing the development, in subsequent ages, of alterations in food habits in their descendants. In this aspect, leptin can be particularly effective when administered to women during the third trimester of pregnancy.

On the other hand, by "preparation for consumption" it is understood any preparation/pharmaceutical composition, food composition or nutritional supplement administered or ingested orally.

In one particular embodiment of the invention, for its administration in the prevention and/or treatment of alterations in food habits, leptin is formulated in an appropriate pharmaceutical composition, in the therapeutically effective amount, together with one or more vehicles, adjuvants or pharmaceutically acceptable excipients.

The pharmaceutical composition will be administered orally, either in solid or liquid form. Illustrative examples of pharmaceutical forms for oral administration include tablets, capsules, medicine in the form of granules, solutions, suspensions, etc. and may contain the conventional excipients, such as cohesives, diluents, disintegrators, lubricators, humectants, etc., and may be prepared by conventional methods.

In the particular case in which leptin is supplied to the lactating mother or to the pregnant female, the mode of administration may also be by other ways, such as parenteral or through patches.

In general, the therapeutically effective amount of leptin to administer will depend, among other factors, on the individual to be treated, on the presence of other agents with agonic or antagonic effects, on age, etc. For this reason, the doses mentioned in this invention must be considered only a guide for the expert in the subject, and this person must adjust the doses according to the aforementioned factors. However, leptin, a fragment thereof or a mimetic product of leptin action can be administered one or more times a day, for example, 1, 2, 3 or 4 times a day, in a daily total typical amount comprising between 1 ng and 60 µg per day. In one particular embodiment, the total orally administered dose to a lactating human is approximately 1-2 µg per day during the first month of life, approximately 2-2.5 µg per day during the second month of life and approximately 2.5-3 µg per day from three to five months of life. In the particular case that leptin is supplied to the lactating mother or the pregnant female, the administered amounts could be considerably higher, especially between 0.1 µg and 60 mg, although they may vary depending on the conditions, type of leptin or mimetic and way of administration.

In another particular embodiment, for its administration in the prevention and/or treatment of alterations on food habits, leptin is formulated in an appropriate food composition or in a nutritional supplement, which comprises leptin, a fragment thereof or a mimetic product of leptin action, and a food vehicle. To the effects of the present invention, it is understood by food vehicle any product capable of being used in human or animal feeding or that fits in the definition of food according to current European legislation. The choice of the suitable food vehicle for each case may be carried out by an expert in the subject from the conventional existing food vehicles in the state of the technique.

That food composition may be in the form of a soluble powder, it may be a concentrated liquid, a snack or a ready-to-use formulation suitable for oral intake or for enteral administration. Examples of these compositions may be, among others, a maternal milk, an infant formula milk for lactating neonates or a continuation formula, a dairy product or a derivate such as a milk shake, milk in general (including full-cream milk, semi-skimmed, skimmed, concentrated, pasteurised, flavoured, fermented, soya milk, optionally supplemented with sugars, other carbohydrates, fat and other nutritional additives), a yoghurt, etc.; a juice; a flour product or derivate such as a cake, a bread, a cookie; an oil, sweets, such us chewing gum, candies, etc.

In the case that the food composition is milk, this may come from different mammal species, both humans and, for instance, farm animals and domestic animals. It is not necessary for leptin to come from the same species as the milk, but it is possible for instance to mix leptin from humans with milk from an animal or vice versa.

Typically, the way of mixing leptin and milk will depend on the way that this leptin is going to be administered and it will be selected so as to keep leptin activity in the final mixture. For example, leptin is generally sterilized by thermal treatment, as is the case of pasteurization. However, leptin activity can be seriously affected by this thermal treatment, and in this case it is convenient to add leptin after the sterilization process, or to use bacterial inactivating processes that do not inactivate leptin (radiation, high pressures, etc.).

The daily amount of leptin to administer by means of food composition in any of its forms will be comprised between 1 ng and 60 µg per day. In one particular embodiment, the total administered dose to a lactating mammal is approximately 1-2 µg per day during the first month of life, approximately 2-2.5 µg per day during the second month of life and approximately 2.5-3 µg per day from three to five months of life.

In a preferential embodiment of the present invention, this food composition is a children's food product. In an even more preferential embodiment, the children's product is powdered milk for lactating neonates or liquid maternalized milk for lactating neonates. In another preferential embodiment, this children's product is a paste or a baby food or a preparation of infant formula or of continuation. In these cases, the food composition contains leptin in a concentration between 0.1 and 30 µg/kg of product. In a preferential instance, leptin concentration is 2.5 µg/kg of product or it is designed to supply a daily dose over 25 ng when administered to lactating humans.

In another preferential embodiment, the preparation for consumption is a nutritional supplement. This nutritional supplement may be a liquid composition for consumption, such as syrup or a drink, or a solid composition to consume, such as a tablet, capsule or a reconstituting powder.

In another preferential embodiment, leptin, a fragment thereof or a mimetic product of leptin action, is incorporated on the surface of a solid support, such as a dummy or object, so that, when sucking it, the product can be assimilated and incorporated in this way into the organism.

The amount of leptin in the food composition or in the nutritional supplement that may be ingested by a patient will depend on numerous factors such as the state of the patient, his/her body weight, age, among others. Nevertheless, the suitable amount will have to be prescribed by a specialist and will be adjusted in function of the previously mentioned variables. However, leptin, a fragment thereof or a mimetic product of leptin action, may be administered in several doses, for example from 2 to 8 times a day, in order to administer the daily recommended amount or it may be ingested in one single dose.

On the other hand, leptin, a fragment thereof or a mimetic product of leptin action, may be used together with other products or additional drugs that are useful in the prevention and/or treatment of alterations in food habits. These products or additional drugs may be part of the same pharmaceutical composition, food composition or nutritional supplement or, alternatively, may be provided in the form of a separate composition for simultaneous administration or not to the pharmaceutical composition, food composition or nutritional supplement that comprises leptin, a fragment of leptin or a mimetic product of leptin action.

The following example aims to illustrate the invention but it must not be considered as setting a limit in its scope.

### Examples

### General procedure for the selection of the rat groups to be studied

Neonatal rats from different dams were selected. To obtain the pups, 3-month-old female virgin rats were matched with male rats. After matching, each female was placed in an individual cage with free access to water and standard chow (3000 Kcal/kg). Rats were kept in a room with controlled temperature (22°C) and 12 h light-dark cycle. At day 1 after delivery 10 pups per dam were kept and randomly assigned into 2 groups: control group and leptin-treated group. From day 1 to day 20 of lactation, and during the first 2 h of the beginning of the light cycle, 20 µL of the vehicle (water) or of murine leptin dissolved in water solution were daily and orally administered, using a pipette, to the control and leptin-treated group respectively. The amount of leptin given to animals was progressively increased from 1 ng of leptin on day 1 to 43.8 ng of leptin on day 20; this was calculated in other to supply five times the average amount of the daily leptin intake from maternal milk. On day 21, after weaning, both control and leptin-treated male rats were housed individually and fed on a standard chow diet.

### Example 1. Study of the effect of leptin administration during lactation on food preferences in adulthood

A test [two bottles preference test (Kozak et al., Eur. J. Neurosci., 2005, 21, 2887-92)] was performed to assess the food preferences (carbohydrate-rich or fat-rich diet) in 8-month-old adult rats for the two selected groups as previously described: control rats and rats treated with leptin during the lactating period. This test consists of preparing two liquid meals, one carbohydrate-rich and the other fat-rich, both with the same caloric density (2.31 Kcal/g), and to determine the intake of each diet offered simultaneously for 1 hour.

It was observed that the total food intake consumed during 1 hour (considering both carbohydrate- and fat-rich diet) was similar in control and leptin-treated groups. However, both groups of animals differed in their dietary preferences. Control animals displayed a greater preference for the fat-rich diet than for the carbohydrate-rich diet (* p<0.05, Student's *t* test). On the other hand, leptin-treated rats showed a high preference for the carbohydrate-rich diet (Figure 1).

### Example 2. Effect of the supplementation of leptin during lactation on leptinemia and leptin sensitivity in adulthood.

For this study, control and leptin-treated rats during lactation as described above were used. These rats, from both groups, were subject to 3 different feeding conditions: *ad libitum,* 14 hours fasting, and 3 hours refeeding after 14 h fasting and circulating leptin levels were analysed, afterwards. Figure 2 shows that circulating leptin levels were significantly lower in rats that were treated with leptin during lactation than in their control counterparts.

In addition, rats that were treated with leptin during lactation were more sensitive to the anorexigenic effects of leptin than control animals. This was proved by determining the effects of the intraperitoneal administration of exogenous leptin (compared to the intraperitoneal administration of saline) on food intake of control and leptin-treated rats. Intraperitoenal administration of leptin (2mg/kg) resulted, after 1 h, in a slight, but not significant, decrease in food intake in both groups, which was more pronounced in leptin-treated than in control group (reduction of 23% and 15%, respectively compared to their control injected with saline) (Figure 3). In addition, the anorexic effect of exogenous leptin was more persistent in leptin-treated than in control animals: during the second hour after the intraperitoneal leptin administration, the effect was imperceptible in control animals while it was still evident in leptin-treated rats which showed a 22% lower food intake in this period than their respective controls which received an intraperitoneal administration of saline. Considering the first 12 h after the intraperitoenal administration of leptin (corresponding to the dark period), the anorectic effect of exogenous leptin was significant in leptin-treated rats (p<0.05, Student's *t* test) but not in the control group. No significant effects of exogenous leptin on food intake in either of the two groups were observed when the period of 24 h after the exogenous administration of leptin was considered.

Therefore, the role of leptin supplied during lactation in the control of appetite in further ages and in another important aspect of the control of feeding behaviour in later life, such are food preferences, is demonstrated. The role of leptin supplied during lactation in reducing leptinemia and improving the sensitivity to this hormone is also demonstrated.

## Claims

1. Use of leptin, a fragment thereof or a mimetic product of leptin action, in the production of a preparation for consumption to administer to lactating mammals, in particular lactating humans, for the treatment and/or prevention of alterations in food habits during the adulthood.

2. Use according to claim 1 wherein the alteration in food habits consists of the acquisition of undesirable food preferences.

3. Use according to claim 2 wherein the undesirable food preference is a preference for the intake of fat-rich and high-energy foods, in particular it is a preference for the intake of foods rich in saturated fats.

4. Use according to claim 1 wherein the alteration in food habits consists of an appetite alteration, in particular this alteration is an excess of appetite.

5. Use of leptin, a fragment thereof or a mimetic product of leptin action, in the production of a preparation for consumption to administer to lactating mammals, in particular lactating humans, for the treatment and/or prevention of the deterioration of leptin sensitivity, of a deficient action of endogenous leptin or alterations that are accompanied by a deficient sensitivity or response to the administration of exogenous leptin, in adulthood.

6. Use according to any of claims 1 to 5, in which leptin, a fragment thereof or a mimetic product of leptin action, is administered to lactating humans and lactating domestic animals.

7. Use according to any of claims 1 to 5, in which leptin, a fragment thereof or a mimetic product of leptin action, is also administered to lactating mothers and pregnant females.

8. Use according to any of claims 1 to 7 wherein the preparation for consumption is a pharmaceutical composition.

9. Use according to any of claims 1 to 8 wherein the preparation for consumption is a food, a food composition, or a nutritional supplement.

10. Use according to claim 9 wherein the food composition is a dairy product or derivate thereof, a juice, a flour product or derivate thereof; an oil or a sweet.

11. Use according to claim 10 wherein the dairy product is milk, maternal milk, infant formula milk or a continuation formula.

12. Use according to claim 11 wherein leptin and milk come from the same or from a different mammal.

13. Use according to any of claims 1 to 12, wherein the daily total administered amount of leptin is comprised between 1 ng and 60 µg.

14. Use according to claim 9 wherein the food composition is a children's food product.

15. Use according to claim 14 wherein to children's food product is powdered milk for lactating neonates or liquid maternalized milk for lactating neonates.

16. Use according to claim 14 wherein the children's food product is a paste or a baby food or a preparation of infant formula or of continuation.

17. Use according to claims 15 or 16 wherein the children's food product comprises leptin in a concentration between 0.1 and 30 µg/L of product, preferably leptin concentration is of 2.5 µg/L of product, or is designed to supply a daily dose of leptin over 25 ng when administered to lactating humans.

18. Use according claim 9 wherein the nutritional supplement is a liquid composition for consumption selected from syrup and a drink, or a solid composition for consumption selected from a tablet, a capsule and reconstituting powder.

19. Use according to any of claims 8 to 18, wherein the pharmaceutical composition, the food composition or the nutritional supplement is administered in combination with another additional product useful in the prevention and/or treatment of alterations in food habits.

20. Use according to claim 19 wherein the additional product useful in the prevention and/or treatment of alteration in food habits, is provided in the form of a separate composition for its simultaneous administration or not to the pharmaceutical composition, food composition or nutritional supplement that comprises leptin, a fragment thereof or a mimetic product of leptin action.

21. Method for the treatment and/or prevention of an alteration in food habits in adulthood, this method comprises the administration to a lactating mammal of an effective quantity of leptin, a fragment thereof or a mimetic product of leptin action.

22. Method according to claim 21 wherein the alteration in the food habit consists of the acquisition of food preferences that are considered undesirable.

23. Method according to claim 22 wherein the undesirable food preference is a preference for the intake of fat-rich and high-energy foods, in particular it is a preference for the intake of foods rich in saturated fats.

24. Method according to claim 21 wherein the alteration in the food habit consists of an alteration of the appetite.

25. Method for the treatment and/or prevention of a deterioration in leptin action, of a deficient action of endogenous leptin or of alterations that are accompanied by a deficient sensitivity or response to the administration of exogenous leptin, in the adulthood, this method comprises the administration to a lactating mammal of an effective quantity of leptin, a fragment thereof or a mimetic product of leptin action.
